# EUROPEAN PATENT APPLICATION

(11) **EP 2 774 605 A1**
(43) Date of publication of application: **10.09.2014**
(21) Application number: 13154284.7
(22) Date of filing: 06.02.2013
(51) Int. Cl.: A61K 9/00, A61K 47/26, A61K 9/14, A61K 9/16, A61K 9/20, A61K 9/28

(54) **Pharmaceutical composition comprising almotriptan malate having uniform drug distribution and potency**

(71) Applicant: Galenicum Health S.L., 08005 Barcelona (ES)
(72) Inventor: Forcat Subirana, Teia, 08005 Barcelona (ES)
(74) Representative: Torrejón-Nieto, Javier

(57) **Abstract**

The present invention relates to tablets comprising almotriptan malate and mannitol, wherein the mannitol is in crystalline form and the bulk density of the mannitol is below 0.75 g/ml. The present invention also relates to a specific crystalline form of almotriptan malate characterized by its particle size distribution.

## Description

The present invention relates to a method for manufacturing a pharmaceutical composition having uniform drug distribution and potency including the compositions and medicaments produced therefrom. In particular, it relates to methods and compositions for use in the manufacture of low dosage pharmaceutical compositions in the form of tablets containing mannitol.

### STATE OF THE ART

Migraine is a common chronic condition with an ever-expanding therapeutic armamentarium. As therapeutic options multiply, it is increasingly important to understand patients' attitudes and preferences regarding various treatment characteristics. Several strategies have evolved to establish treatment priorities in migraine, and rationalize and prioritize end points as well as outcomes to meet the needs of patients. A survey of a population-based sample of migraines indicated that an overwhelming majority of patients consider complete relief of head pain, no recurrence, and rapid onset of action as important or very important attributes of acute migraine therapy. An analysis of the relationship between clinical end points and satisfaction found that more than 90% of patients who were pain-free at 2 hours were at least somewhat satisfied with treatment, but satisfaction was dependent on relatively rapid pain relief. Using a "willingness-to-pay" approach, results indicated that while patients will pay more for migraine treatment that produces rapid, consistent relief without adverse effects or recurrence, speed of complete relief is the most valued attribute. By assessing physician preferences and practices, degree of pain relief and rapid onset were identified as the most important attributes of acute therapy. Based on results from preference studies of triptans, 50 % of patients cited more rapid pain relief as the most important determinant of treatment preference. Based on these various approaches, the consensus view is that both clinicians and patients desire a broad range of positive migraine treatment attributes, but rapid onset of complete pain relief is a particularly important priority.

Triptans are a family of tryptamine-based drugs used as abortive medication in the treatment of migraines and cluster headaches. They were first introduced in the 1990s. Their action is attributed to their agonist effects on serotonin 5-HT1B and 5-HT1 D receptors in cranial blood vessels (causing their constriction) and subsequent inhibition of pro-inflammatory neuropeptide release. Evidence is accumulating that these drugs are effective because they act on serotonin receptors in nerve endings as well as the blood vessels. This leads to a decrease in the release of several peptides, including CGRP and substance P.

Almotriptan is selective agonist for vascular serotonin (5-HT) receptor subtype, causing vasoconstriction of cranial arteries, as disclosed in the ES 2 056 025 B1. Its chemical name is N,N-dimethyl-2-[5-(pyrrolidin-1-ylsulfonylmethyl)- 1H-indol-3-yl]-ethanamine and the structural formula is (I):

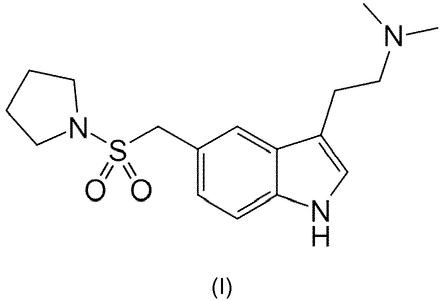

Almotriptan malate is a potent compound thus requiring special handling to reduce operator exposure during the manufacturing process.

An important unit operation in the production of a pharmaceutical solid dosage form is the mixing of the powder constituents. One of the primary objectives of the mixing process is to obtain a homogeneous mixing of the active pharmaceutical ingredient (API) in the formulation. Demonstrating homogeneity ensures that the unit dose formulation contains active ingredient(s) uniformly distributed throughout the formulation; thereby the active ingredient is being administered in the appropriate amount.

Blend uniformity is a function of both the formulation and mixing action. Once the formulation is optimized from a theoretical process standpoint, blend uniformity must be validated during piloting and scale-up. From a manufacturer's perspectives, poor uniformity generates unacceptable amounts of discarded products, resulting in significant loss of revenue. It is insufficient to show that adequate distribution of the drug is obtained in the final product, it must be demonstrated within the blend also. The FDA Guidelines state that the USP criteria for content uniformity is 85-115 % but the industry standard for content uniformity is 90-110 %.

The homogeneity is normally determined by removing a small number of powder samples from the powder mixer with a probe thief and sending the samples to a quality control laboratory distant from the process line for, e.g. high pressure liquid chromatography (HPLC) analysis. The API content in the samples and the relative standard deviation (RSD) of the API content between the samples is then obtained and compared to specified values in order to decide wherever the batch is sufficiently homogeneous.

Of particular concern is the lack or loss of homogeneity of the powders during the blending steps. Although one can improve the equipment and conditions where the mixing takes places to have a good mixing, it is desired to have a powder which does not depend on the equipments used for its manufacture nor on the manufacturing conditions to have a good mixing. In addition, it is important that not only the powders are homogenous, but the final compositions are stable and show an immediate release dissolution profile. Therefore, there is a need for an improved formulation and process that would minimize demix of active ingredients during the manufacture or the storage of medicaments, in particular, those having a low dosage content, whereas in turn obtaining compositions which are stable and have a fast onset.

### SUMMARY OF THE INVENTION

The present invention relates to a pharmaceutical composition having high uniform drug distribution and potency, which at the same time are stable and have a fast onset. The present invention also provides a process for maintaining uniformity and potency during the manufacture of a pharmaceutical composition containing an active ingredient. The process includes means for reducing the loss of active ingredients during the manufacturing process of a pharmaceutical composition or medicament.

Uniform distribution of each component in a powder mixture is desired to assure uniform subdivision of the individual components when the powder mixture is subdivided. For example, compression of granules of a drug product requires good uniformity of the drug together with the excipients in the granulation so that each tablet has the desired dose. Uniformity of a powder mixture can be compromised post-mixing during storage and handling of powders. For example, vibration in the storage bins due to the operation of large-scale equipment can lead to segregation of a uniform mixture of the components with differences in particle size and/or density. Segregation can also happen during material transfer. For example, flow of a powder blend through a hopper from a closed chamber can result in a countercurrent flow of air, which can partially fluidize the powder, leading to a segregation based on the fluidization potential of the different components.

When developing pharmaceutical compositions, it is not only important that the powders are uniform, but that the final compositions are stable and have the desired dissolution profile.

In a first aspect of the present invention, it is provided a pharmaceutical composition in the form of an immediate release tablet comprising almotriptan malate and mannitol, wherein the mannitol is in crystalline form and the bulk density of the mannitol is below 0.75 g/ml.

In a second aspect of the present invention, it is provided a pharmaceutical powder comprising almotriptan malate and mannitol, wherein the mannitol is in crystalline form and the bulk density of the mannitol is below 0.75 g/ml. The present inventor has identified that the powders as herein disclosed can be mixed homogeneously and the homogeneity of the powders is stable. Optimum mixing is a prerequisite for manufacturing of all solid dosage forms that involves powder mixing and it has a critical contribution in achieving uniformity of content. Also, having powders with good characteristics and behaviour is essential to control the manufacturing of pharmaceutical compositions. Mixing is defined as shuffling type unit operation process involving both large and small particle groups and even individual particles. Mixing is an energy consuming process that produces a random distribution of particles. It is dependent on the probability that an event happens in a given time and once the desired mixing has been attained, it is essential that the particles in the mix cease movement so that the system may exist in a state of static equilibrium without segregation.

In response to the concerns by ANDA applicants regarding inconsistency review in chemist's recommendations, the US FDA published draft guidance in August 1999 for blend uniformity analysis. The FDA indicated that there is a strong need for routine blend uniformity testing following process validation to ensure content uniformity within the blend. It is insufficient to show that adequate distribution of the drug is obtained in the final product; it must be demonstrated within the blend also. This guidance gives the basics of the powder mix and the representative sample. When the sample is based on thief blend, if the % RSD is less than 1, then the sample passes the test. The FDA guidelines state that the USP criteria for content uniformity are 85-115 %. But the industry standard for the blend uniformity is 90-110 %. Following the FDA guidelines makes the validation process difficult. So to make the task easy for validation, a range of 90-110 % is necessary.

In a third aspect of the present invention, it is provided a granulate comprising almotriptan malate and mannitol, wherein the mannitol is in crystalline form and the bulk density of the mannitol used for the manufacture the granules is below 0.75 g/ml.

In a fourth aspect of the present invention, it is provided a almotriptan malate having X-Ray powder diffraction pattern with peaks at 15.4, 16.2 and 22.1° 2θ (±0.2° 2θ) and wherein the D50 of the almotriptan malate is higher than 20 microns and the D90 is higher than 50 microns.

In a fifth aspect of the present invention, it is provided a pharmaceutical batch of at least 10,000 units of the pharmaceutical composition of the first aspect.

In a sixth aspect of the present invention, it is provided a cardboard box with a patient information leaflet in pack sizes of 2, 3, 4, 6, 7, 9, 12, 14 or 18 units of the pharmaceutical composition of the first aspect, wherein the units are packaged in blisters, and more preferably the blister is an aluminium/aluminium blister or an aluminium/PVC blister.

In a seventh aspect of the present invention, it is provided processes for the preparation of the pharmaceutical composition of the first aspect.

### DETAILED DESCRIPTION OF THE INVENTION

In a further embodiment of the pharmaceutical composition as herein disclosed, the bulk density of the mannitol ranges from 0.40 to 0.67 g/ml, preferably from 0.48 to 0.62 g/ml, more preferably from about 0.52 to about 0.58 g/ml, and even more preferably is about 0.55 g/ml.

Several mannitols are available in the market: Pearlitol® 110 SD having a mean diameter 100 microns, Pearlitol® 200 SD having a mean diameter 180 microns, Pearlitol® 160C having a mean diameter 160 microns, Pearlitol® 50 C having a mean diameter 50 microns, Pearlitol® 25 C having a mean diameter 25 microns, Pearlitol® 300 DC having a mean diameter 250 microns, Pearlitol® 400 DC having a mean diameter 360 microns and Pearlitol 500® DC having a diameter 520 microns.

The bulk density of a powder is the ratio of the mass of an untapped powder sample and its volume including the contribution of the interparticulate void volume. Hence, the bulk density depends on both the density of powder particles and the spatial arrangement of particles in the powder bed. The bulk density is expressed in grams per ml (g/ml). The bulk density of a powder is determined by measuring the volume of a known weight of powder sample into a graduated cylinder. The bulk density of the mannitol is measured according to the Second Supplement to USP 35-NF 30 Physical Tests / <616> Bulk Density and Tapped Density of Powders 5637 (Method I).

In a further embodiment of the pharmaceutical composition as herein disclosed, the mannitol has a mean particle size measured by laser light diffraction from 15 to 180 microns, preferably from 35 to 80 microns.

In a further embodiment of the pharmaceutical composition as herein disclosed, the bulk density of the mannitol ranges from 0.40 to 0.67 g/ml and the mannitol has a mean particle size measured by laser light diffraction from 15 to 180 microns.

In a further embodiment of the pharmaceutical composition as herein disclosed, the bulk density of the mannitol ranges from 0.48 to 0.62 g/ml and the mannitol has a mean particle size measured by laser light diffraction from 15 to 180 microns.

In a further embodiment of the pharmaceutical composition as herein disclosed, the bulk density of the mannitol ranges from 0.48 to 0.62 g/ml and the mannitol has a mean particle size measured by laser light diffraction from 35 to 80 microns.

In a further embodiment of the pharmaceutical composition as herein disclosed, the bulk density of the mannitol ranges from about 0.52 to about 0.58 g/ml, preferably is about 0.55 g/ml, and the mannitol has a mean particle size measured by laser light diffraction from 35 to 80 microns.

Laser diffraction is a particle size analysis method that uses the average relative angular intensity of scattered light. Instruments that use laser light diffraction to measure particle size have been available for many years from different manufacturers (see e.g. US 5,610,712).

The particle size determination of the product is determined according to the following method: Apparatus: Malvern Mastersizer 2000S with a sample dispersion unit. Dispersant: light liquid paraffin. Instrument preparation: the background detect signal should be less than 100, and obscuration % value should within the range of 10 - 20 %. Sample preparation: weigh about 150 mg of sample and add directly into 100 ml beaker that has been previously filled with the dispersant. Ultrasonic for 3 minutes. Pipette prepared sample into measuring cell.

In a further embodiment of the pharmaceutical composition as herein disclosed, the almotriptan malate is non-micronized, preferably a non-micronized crystalline form. One advantage of the process as herein disclosed is that does not require the micronization of the almotriptan maleate, which simplifies the process.

Almotriptan malate refers to almotriptan D,L-hydrogen malate.

The pharmaceutical compositions as herein disclosed are oral immediate release dosage forms. An immediate release dosage form as herein disclosed has to be understood as a dosage form having a dissolution performance such as 60 % or more of the agent contained in said pharmaceutical composition dissolves within 60 minutes. In a preferred embodiment, the immediate release composition as herein disclosed releases at least 80 % of the agent in 60 minutes. In another embodiment, the pharmaceutical composition as herein disclosed releases at least an 80 % of the agent in 45 minutes, preferably in 35 minutes and more preferably in 30 minutes. In another embodiment, the pharmaceutical composition as herein disclosed releases at least an 80 % of the agent in 30 minutes and at least a 95 % of the agent in 60 minutes. In a most preferred embodiment, the pharmaceutical composition as herein disclosed releases at least an 80 % of the agent in 15 minutes, and at least a 95 % of the agent in 60 minutes. The dissolution test for an immediate release pharmaceutical composition comprising the agent as herein disclosed is performed in the following conditions: USP Apparatus: II (Paddles). Speed 50 rpm. Medium: potassium dihydrogen phosphate buffer, pH 6.8. Wavelength: 228 nm. Temperature: 37 ± 0.5°C.

The term "stable" as used herein refers to a pharmaceutical composition comprising almotriptan malate wherein the total content of impurities originating from the decomposition of almotriptan malate does not exceed 5 % area by HPLC at 228 nm, preferably 3 % area, more preferably 2 % area and most preferably 1 % area determined by liquid chromatography at 228 nm if such a composition is stored for 2 months at 40 °C and 75 % relative humidity (RH). The pharmaceutical composition and the process as herein disclosed provide such a stable almotriptan maleate pharmaceutical composition, that the impurity almotriptan monomethyl analog whose chemical name is N-Methyl-2-(5-((pyrrolidin-1-ylsulfonyl)methyl)-1H-indol-3-yl) ethanamine oxalate is well below 0.1 %.

In a further embodiment of the pharmaceutical composition as herein disclosed, the D50 of the almotriptan malate is higher than 20 microns, preferably is less than 250 microns, more preferably is between 30 and 190 microns, even more preferably is between 50 and 150 microns, and even more preferably is between 65 and 135 microns, and yet even more preferably is between 80 and 115 microns.

In a further embodiment of the pharmaceutical composition as herein disclosed, the D90 of the almotriptan malate is higher than 50 microns, preferably is less than 700 microns, more preferably is between 80 and 550 microns.

In a further embodiment of the pharmaceutical composition as herein disclosed, the D90 of the almotriptan malate is between 110 and 475 microns, preferably is between 150 and 390 microns, more preferably is between 200 and 300 microns.

In a further embodiment of the pharmaceutical composition as herein disclosed, the D50 of the almotriptan malate is higher than 20 microns and the D90 of the almotriptan malate is higher than 50 microns.

In a further embodiment of the pharmaceutical composition as herein disclosed, the D50 of the almotriptan malate is less than 250 microns and the D90 of the almotriptan malate is less than 700 microns.

In a further embodiment of the pharmaceutical composition as herein disclosed, the D50 of the almotriptan malate is between 30 and 190 microns and the D90 of the almotriptan malate is between 80 and 550 microns.

In a further embodiment of the pharmaceutical composition as herein disclosed, the D50 of the almotriptan malate is between 50 and 150 microns and the D90 of the almotriptan malate is between 110 and 475 microns.

In a further embodiment of the pharmaceutical composition as herein disclosed, the D50 of the almotriptan malate is between 65 and 135 microns and the D90 of the almotriptan malate is between 150 and 390 microns.

In a further embodiment of the pharmaceutical composition as herein disclosed, the D50 of the almotriptan malate is between 80 and 115 microns the D90 of the almotriptan malate is between 200 and 300 microns.

D50 and D90 represent the 50 percentile and the 90 percentile of the particle size volume distribution. That is, D50 (D90) is a value on the distribution such that 50 % (90 %) of the particles has a particle size of this value or less.

In a further embodiment of the pharmaceutical composition as herein disclosed, the almotriptan malate is in crystalline form.

In a further embodiment of the pharmaceutical composition as herein disclosed, almotriptan malate shows an X-Ray powder diffraction pattern with peaks at 15.4, 16.2 and 22.1° 2θ (±0.2° 2θ).

In a further embodiment of the pharmaceutical composition as herein disclosed, the D50 of the almotriptan malate is higher than 20 microns, the D90 of the almotriptan malate is higher than 50 microns and shows an X-Ray powder diffraction pattern with peaks at 15.4, 16.2 and 22.1° 2θ (±0.2° 2θ).

In a further embodiment of the pharmaceutical composition as herein disclosed, the crystalline form of almotriptan malate has an X-Ray powder diffraction pattern with peaks at 10.7, 15.4, 16.2, 18.3, 19.6 and 22.1° 2θ (±0.2° 2θ).

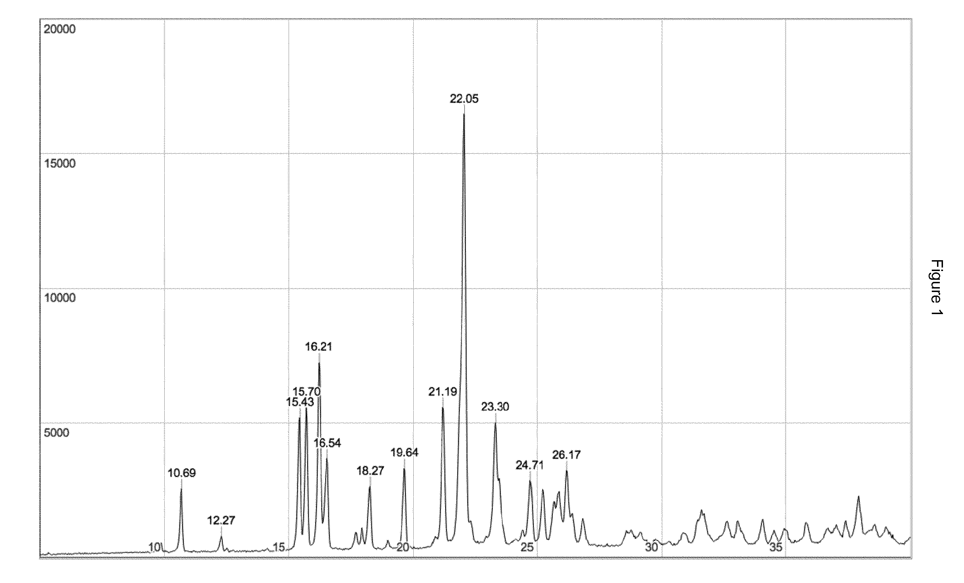

In a further embodiment of the pharmaceutical composition as herein disclosed, the crystalline form of almotriptan malate has an X-Ray powder diffraction pattern as depicted in figure 1. X-Ray Powder Diffraction was performed as explained in the examples.

In a further embodiment of the pharmaceutical composition as herein disclosed, the ratio by weight of (almotriptan malate):(mannitol) is from 1:1 to 1:10.

In a further embodiment of the pharmaceutical composition as herein disclosed, the ratio by weight of (almotriptan malate):(mannitol) is from 1:1 to 1:10, the D50 of the almotriptan malate is higher than 20 microns, the D90 of the almotriptan malate is higher than 50 microns and shows an X-Ray powder diffraction pattern with peaks at 15.4, 16.2 and 22.1° 2θ (±0.2° 2θ).

In a further embodiment of the pharmaceutical composition as herein disclosed, the ratio by weight of (almotriptan malate):(mannitol) is from 1:1 to 1:10, the bulk density of the mannitol ranges from 0.40 to 0.67 g/ml and the mannitol has a mean particle size measured by laser light diffraction from 15 to 180 microns.

In a further embodiment of the pharmaceutical composition as herein disclosed, the ratio by weight of (almotriptan malate):(mannitol) is from 1:1 to 1:10, the D50 of the almotriptan malate is higher than 20 microns, the D90 of the almotriptan malate is higher than 50 microns, shows an X-Ray powder diffraction pattern with peaks at 15.4, 16.2 and 22.1° 2θ (±0.2° 2θ), the bulk density of the mannitol ranges from 0.40 to 0.67 g/ml and the mannitol has a mean particle size measured by laser light diffraction from 15 to 180 microns.

In a further embodiment of the pharmaceutical composition as herein disclosed, the ratio by weight of (almotriptan malate):(mannitol) is from 1:2 to 1:7, preferably is from 1:3 to 1:4.5, more preferably is from 1:3.5 to 1:4.0.

In a further embodiment of the pharmaceutical composition as herein disclosed, the pharmaceutical composition further comprises at least a diluent, preferably in an amount from 2 to 20 % w/w in respect of the total amount of the pharmaceutical composition, more preferably from 7 to 15 % w/w.

In a further embodiment of the pharmaceutical composition as herein disclosed, the pharmaceutical composition further comprises microcrystalline cellulose, preferably in an amount from 2 to 20 % w/w in respect of the total amount of the pharmaceutical composition, more preferably from 7 to 15 % w/w.

The term "diluent" refers to an agent that dilutes the substance or solution to which it is added.

In a further embodiment of the pharmaceutical composition as herein disclosed, the pharmaceutical composition further comprises at least a lubricant, preferably in an amount from 0.1 to 3 % w/w in respect of the total amount of the pharmaceutical composition, more preferably from 0.5 to 1.5 % w/w.

In a further embodiment of the pharmaceutical composition as herein disclosed, the pharmaceutical composition further comprises sodium stearyl fumarate, preferably in an amount from 0.1 to 3 % w/w in respect of the total amount of the pharmaceutical composition, more preferably from 0.5 to 1.5 % w/w.

As used herein, "lubricant" means a substance that reduces friction between the composition of the present invention and the surfaces of the apparatus used to compact the composition into a compressed form.

In a further embodiment of the pharmaceutical composition as herein disclosed, the pharmaceutical composition further comprises at least a binder, preferably in an amount from 1 to 8 % w/w in respect of the total amount of the pharmaceutical composition, more preferably from 2 to 5 % w/w.

In a further embodiment of the pharmaceutical composition as herein disclosed, the pharmaceutical composition further comprises polyvinylpyrrolidone, preferably in an amount from 1 to 8 % w/w in respect of the total amount of the pharmaceutical composition, more preferably from 2 to 5 % w/w

The term "binder" refers to a pharmaceutically acceptable substance or mixture of pharmaceutically acceptable substances that provides or improves the cohesiveness of the granules and can also contribute to the cohesiveness of the compressed tablets

In a further embodiment of the pharmaceutical composition as herein disclosed, the pharmaceutical composition further comprises at least a glidant, preferably in an amount from 2 to 8 % w/w in respect of the total amount of the pharmaceutical composition, more preferably from 3 to 6 % w/w in respect of the total amount of the pharmaceutical composition.

In a further embodiment of the pharmaceutical composition as herein disclosed, the pharmaceutical composition further comprises sodium carboxymethyl starch, preferably in an amount from 2 to 8 % w/w in respect of the total amount of the pharmaceutical composition, more preferably from 3 to 6 % w/w in respect of the total amount of the pharmaceutical composition.

As used herein, the term "glidant" refers to a pharmaceutically acceptable material that, when added to a dried granulated pharmaceutical composition in a sufficient amount, enhances the flow of the granules mixtures by reducing interparticle friction during processing.

In a further embodiment of the pharmaceutical composition as herein disclosed, the tablet is a film coated tablet, preferably the amount of film coating ranges from 1 to 5 % w/w.

In a further embodiment of the pharmaceutical composition as herein disclosed, the water content of the pharmaceutical composition is lower than 4 % w/w measured by loss on drying. Preferably, the water content of the pharmaceutical composition is lower than 2 % w/w measured by loss on drying.

In a further embodiment of the pharmaceutical composition as herein disclosed, the tablet has a hardness of less than 70 N, preferably the tablet has a hardness from 30 to 55 N and more preferably the tablet has a hardness from 35 to 45 N. The pharmaceutical composition as herein disclosed provides good dissolution profiles despite such values of hardness.

Tablets must be able to withstand the rigors of handling and transportation experienced in the manufacturing plant, in the drug distribution system, and in the field at the hands of the end users (patients/consumers). Manufacturing processes such as coating, packaging, and printing can involve considerable stresses, which the tablets must be able to withstand. For these reasons, the mechanical strength of tablets is of considerable importance and is routinely measured. Tablet strength serves both as a criterion by which to guide product development and as a quality control specification. One commonly employed test of the mechanical integrity of tablets is their breaking force or hardness, which is the force required to cause them to fail (i.e., break) in a specific plane.

In a further embodiment, the pharmaceutical composition as herein disclosed is packaged in a blister. In a preferred embodiment, the blister is an aluminium/PVC blister. In a more preferred embodiment, the blister consists of an aluminium/PVC blister.

In a further embodiment, the pharmaceutical composition as herein disclosed is packaged in a blister, preferably the blister is an aluminium/PVC blister, wherein the pharmaceutical composition is characterized in that the ratio by weight of (almotriptan malate):(mannitol) is from 1:1 to 1:10, the bulk density of the mannitol ranges from 0.40 to 0.67 g/ml and the mannitol has a mean particle size measured by laser light diffraction from 15 to 180 microns.

In a further embodiment, the pharmaceutical composition as herein disclosed is packaged in a blister, preferably the blister is an aluminium/PVC blister, wherein the pharmaceutical composition is characterized in that the ratio by weight of (almotriptan malate):(mannitol) is from 1:1 to 1:10, the D50 of the almotriptan malate is higher than 20 microns, the D90 of the almotriptan malate is higher than 50 microns, shows an X-Ray powder diffraction pattern with peaks at 15.4, 16.2 and 22.1° 2θ (±0.2° 2θ), the bulk density of the mannitol ranges from 0.40 to 0.67 g/ml and the mannitol has a mean particle size measured by laser light diffraction from 15 to 180 microns.

In a further embodiment of the powders as herein disclosed, the ratio by weight of (almotriptan malate):(mannitol) is from 1:1 to 1:10, preferably is from 1:2 to 1:7, more preferably is from 1:3 to 1:4.5, and even more preferably is from 1:3.5 to 1:4.0.

In a further embodiment of the powders as herein disclosed, the pharmaceutical powder is uniformly distributed.

As used herein, the term "uniform distribution" refers to a blended mixture which meets the FDA criteria (Guidance for Industry ANDA's: Blend Uniformity Analysis, published August 1999) of 10 individual blend samples achieving 90-110% potency of the theoretical strength with an RSD of <5% for all blend samples.

The term "uniform potency" refers to a blended mixture that maintains a drug substance activity level greater than or equal to about 90% throughout the manufacturing process.

The phrase "pharmaceutically acceptable" indicates that the substance or composition must be compatible chemically and/or toxicologically, with the other ingredients comprising a formulation, and/or the mammal being treated therewith.

The term "active ingredient" refers to a therapeutically active compound, as well as any prodrugs thereof and pharmaceutically acceptable salts, hydrates and solvates of the compound and the prodrugs.

The term "appropriate period of time" or "suitable period of time" refers to the period of time necessary to achieve a desired effect or result. For example, a mixture may be blended until a uniform distribution is reached that is within an acceptable qualitative range for a given application or use of the blended mixture.

In a further embodiment, almotriptan malate has an X-Ray powder diffraction pattern comprising at least the peaks 15.4, 16.2 and 22.1 2θ (±0.2° 2θ) and the D50 of the almotriptan malate is higher than 20 microns and the D90 is higher than 50 microns and the almotriptan malate is non-micronized.

It is also provided the use of the crystalline form of almotriptan malate as disclosed herein in a process for the manufacture of immediate release tablets.

In a further embodiment, it is provided the use of the almotriptan malate having an X-Ray powder diffraction pattern comprising at least the peaks 15.4, 16.2 and 22.1° 2θ (±0.2° 2θ) and wherein the D50 of the almotriptan malate is higher than 20 microns and the D90 is higher than 50 microns for the manufacture of immediate release tablets, preferably wherein the manufacturing process does not comprise the micronization of the almotriptan malate.

In a further embodiment, it is provided a pharmaceutical batch wherein the amount of almotriptan malate in each unit dose is from about 17.0 to about 18.0 mg.

In a further embodiment of the pharmaceutical batch as herein disclosed, the amount of almotriptan malate in each unit dose from about 17.0 to about 18.0 mg, wherein the units are packaged in blisters, preferably the blister is an aluminium/aluminium blister or an aluminium/PVC blister, although a aluminium/PVC blister is less protective than an aluminium/aluminium blister. In a more preferred embodiment, the blister consists of an aluminium/PVC blister.

The term "blister" or "bubble pack" refers to a sheet in a package construction with recesses designed to hold dosage forms. The sheet may be a plastic film, foil, or combination thereof. Normally, a blister is a product consisting of a flat structure in which blisters are formed, generally by means of a heating process or blowing air, into which the single elements to be packaged are inserted. The blisters are then hermetically sealed using flat strips of appropriate thermomoldable materials (plastics, aluminium, paper), which represent the frangible element through which it is then possible to remove the product. The primary component of a blister pack is a cavity or pocket made from a formable web, usually a thermoformed plastic. This usually has a backing of paperboard or a lidding seal of aluminum foil or plastic. An aluminium/PVC blister refers to a blister where the thermomoldable material is from PVC and the backing is a lidding seal of aluminium foil. Blister packs are commonly used as unit-dose packaging for pharmaceutical tablets, capsules or lozenges. Blister packs can provide barrier protection for shelf life requirements, and a degree of tamper resistance. In the USA, blister packs are mainly used for packing physician samples of drug products, or for Over The Counter (OTC) products in the pharmacy. In other parts of the world, blister packs are the main packaging type since pharmacy dispensing and re-packaging are not common. A series of blister cavities is sometimes called a blister card or blister strip as well as blister pack. The difference between a strip pack and blister pack is that a strip pack does not have thermo-formed or cold formed cavities; the strip pack is formed around the tablet at a time when it is dropped to the sealing area between sealing moulds. In some parts of the world the pharmaceutical blister pack is known as a Push-Through-Pack (PTP), an accurate description of two key properties (i) the lidding foil is brittle allowing to press the product out while breaking the lidding foil and (ii) a semi-rigid formed cavity being sufficiently collapsable to be able to dispense the tablet or capsule by means of pressing it out with your thumb. The main advantages of unit-dose blister packs over other methods of packaging pharmaceutical products are the assurance of product/packaging integrity (including shelf life) of each individual dose and the possibility to create a compliance pack or calendar pack by printing the days of the week above each dose. Blister packs can be created by means of a form-fill-seal process at the pharmaceutical company or designated contract packer. A form-fill-seal process means that the blister pack is created from rolls of flat sheet or film, filled with the pharmaceutical product and closed (sealed) on the same equipment. Such equipment is called a blisterline. There are two types of blister machine's design: rotary and flat-plate.

As used herein, the term "unit dose" or "unit dosage" refers to a physically discrete unit that contains a predetermined quantity of active ingredient calculated to produce a desired therapeutic effect. The unit dose or unit dosage may be in the form of a tablet, capsule, sachet, etc. referred to herein as a "unit dosage form."

In a further embodiment of the pharmaceutical batch as herein disclosed, the relative standard deviation (RSD) value of content uniformity is less than 5 %, preferably less than 1 %.

In a further aspect of the present invention, it is provided a process for the preparation of a pharmaceutical composition comprising almotriptan malate as herein disclosed by wet granulation.

In a further aspect of the present invention, it is provided a process for the preparation of a pharmaceutical composition as herein disclosed by direct compression.

In a further aspect of the present invention, it is provided a process for the preparation of a pharmaceutical composition as herein disclosed by dry granulation.

In a further aspect of the present invention, it is provided a process for the preparation of a pharmaceutical composition as herein disclosed comprising at least the following step:
i) forming an uniformly distributed powder blend comprising at least almotriptan malate and mannitol, wherein the mannitol is in crystalline form and the bulk density of the mannitol is below 0.75 g/ml.

In a further embodiment, the process further comprises the following step:
ii) wet granulating of the powder blend with a mixture of the binder and water to form granules.

In a further embodiment, the process further comprises the following step:
iii) drying the granules to remove the volatiles, preferably until the water content of the granules is below 2.5 % w/w measured by loss on drying.

In a further embodiment, the process further comprises the following step:
iv) blending the granules with the extragranular excipients, preferably a diluent and a glidant.

In a further embodiment, the process further comprises the following step:
v) lubricating the granulate.

In a further embodiment, the process further comprises the following step:
vi) compressing the uniformly distributed powder or the granules into a tablet form.

In a further embodiment, the process comprises the following steps:
i) forming an uniformly distributed powder blend comprising at least almotriptan malate and mannitol, wherein the mannitol is in crystalline form and the bulk density of the mannitol is below 0.75 g/ml.
ii) wet granulating of the powder blend with a mixture of the binder and water to form granules.
iii) drying the granules to remove the volatiles, preferably until the water content of the granules is below 2.5 % w/w measured by loss on drying.
iv) blending the granules with the extragranular excipients, preferably a diluent and a glidant.
v) lubricating the granulate.
vi) compressing the uniformly distributed powder or the granules into a tablet form.

The amount of water of the pharmaceutical compositions as herein disclosed was measured performing a loss on drying (LOD) using an IR halogen. For the determination of the loss on drying by infrared light the following device was used. Under GMP conditions the measurement was performed using the heat balance MA 45 Moisture Analyzer® (Sartorius AG, Göttingen, Germany). The temperature was set to 105 °C. 1 gram of sample was dried for 30 minutes.

In a further embodiment, the pharmaceutical compositions as herein disclosed comprises the following ratio of components:

| | ratio w/w in respect of the almotriptan D,L-hydrogen malate |
|---|---|
| Almotriptan D,L-hydrogen malate | 1 |
| Mannitol | 1:2 to 1:7 |
| Additional diluent | 1:5 to 1:0.1 |
| Binder | 1:0.05 to 1:0.6 |
| Lubricant | 1:0.01 to 1:0.3 |

The term "batch" as used herein refers to a specific quantity of a drug or other material that is intended to have uniform character and quality, within specified limits, and is produced according to a single manufacturing order during the same cycle of manufacture. A batch, in the case of a drug product produced by continuous process, is a specific identified amount produced in a unit of time or quantity in a manner that assures its having uniform character and quality within specified limits (Code of Federal Regulations Title 21, Food and Drug Administration department of Health and Human Services, Subchapter C, Section 210.3 (b) (2) and (10)).

The term "pharmaceutical batch" as used herein refers to a batch as defined above of a pharmaceutical composition manufactured in accordance with the principles and guidelines of Good Manufacturing Practice (GMP) at an industrial scale and which is intended for commercialization (Directive 91/356/EEC).

Each pharmaceutical batch of finished product must fulfil the regulatory requirements of the corresponding Medicine Agency before being released for sale or supply, such as impurities thresholds and stability data.

The pharmaceutical composition may be manufactured at laboratory scale, not necessarily following GMP and not intended for commercialization. The pharmaceutical composition may also be manufactured for validation, following GMP. A batch of a pharmaceutical composition that is manufactured for validation is called "pilot batch".

Further aspect and embodiments of the present invention can be found in the following numbered clauses:
Clause 1 .- A pharmaceutical composition in the form of an immediate release tablet comprising almotriptan malate and mannitol, wherein the mannitol is in crystalline form and the bulk density of the mannitol is below 0.75 g/ml.
Clause 2 .- The pharmaceutical composition according to the preceding clause, wherein the bulk density of the mannitol ranges from 0.40 to 0.67 g/ml.
Clause 3 .- The pharmaceutical composition according to any one of the preceding clauses, wherein the bulk density of the mannitol ranges from 0.48 to 0.62 g/ml.
Clause 4 .- The pharmaceutical composition according to any one of the preceding clauses, wherein the bulk density of the mannitol ranges from about 0.52 to about 0.58 g/ml, preferably is about 0.55 g/ml.
Clause 5.- The pharmaceutical composition according to any one of the preceding clauses, wherein the mannitol has a mean particle size measured by laser light diffraction from 15 to 180 microns.
Clause 6.- The pharmaceutical composition according to any one of the preceding clauses, wherein the mannitol has a mean particle size measured by laser light diffraction from 35 to 80 microns.
Clause 7.- The pharmaceutical composition according to any one of the preceding clauses, wherein the almotriptan malate is non-micronized, preferably a non-micronized crystalline form.
Clause 8.- The pharmaceutical composition according to any one of the preceding clauses, wherein the D50 of the almotriptan malate is higher than 20 microns.
Clause 9.- The pharmaceutical composition according to any one of the preceding clauses, wherein the D50 of the almotriptan malate is less than 250 microns.
Clause 10.- The pharmaceutical composition according to any one of the preceding clauses, wherein the D50 of the almotriptan malate is between 30 and 190 microns.
Clause 11.- The pharmaceutical composition according to any one of the preceding clauses, wherein the D50 of the almotriptan malate is between 50 and 150 microns.
Clause 12.- The pharmaceutical composition according to any one of the preceding clauses, wherein the D50 of the almotriptan malate is between 65 and 135 microns.
Clause 13.- The pharmaceutical composition according to any one of the preceding clauses, wherein the D50 of the almotriptan malate is between 80 and 115 microns.
Clause 14.- The pharmaceutical composition according to any one of the preceding clauses, wherein the D90 of the almotriptan malate is higher than 50 microns.
Clause 15.- The pharmaceutical composition according to any one of the preceding clauses, wherein the D90 of the almotriptan malate is less than 700 microns.
Clause 16.- The pharmaceutical composition according to any one of the preceding clauses, wherein the D90 of the almotriptan malate is between 80 and 550 microns.
Clause 17.- The pharmaceutical composition according to any one of the preceding clauses, wherein the D90 of the almotriptan malate is between 110 and 475 microns.
Clause 18.- The pharmaceutical composition according to any one of the preceding clauses, wherein the D90 of the almotriptan malate is between 150 and 390 microns.
Clause 19.- The pharmaceutical composition according to any one of the preceding clauses, wherein the D90 of the almotriptan malate is between 200 and 300 microns.
Clause 20.- The pharmaceutical composition according to any one of the preceding clauses, wherein the almotriptan malate is in crystalline form.
Clause 21.- The pharmaceutical composition according the preceding clause, wherein the crystalline form of almotriptan malate shows an X-Ray powder diffraction pattern with peaks at 15.4, 16.2 and 22.1 ° 2θ (±0.2 ° 2θ).
Clause 22.- The pharmaceutical composition according the preceding clause, wherein the crystalline form of almotriptan malate shows an X-Ray powder diffraction pattern with peaks at 10.7, 15.4, 16.2, 18.3, 19.6 and 22.1 ° 2Θ (±0.2 ° 2θ).
Clause 23.- The pharmaceutical composition according to any one of the 3 preceding clauses, wherein the crystalline form of almotriptan malate has an X-Ray powder diffraction pattern as depicted in figure 1.
Clause 24.- The pharmaceutical composition according to any one of the preceding clauses, wherein the ratio by weight of (almotriptan malate):(mannitol) is from 1:1 to 1:10.
Clause 25.- The pharmaceutical composition according to any one of the preceding clauses, wherein the ratio by weight of (almotriptan malate):(mannitol) is from 1:2 to 1:7.
Clause 26.- The pharmaceutical composition according to any one of the preceding clauses, wherein the ratio by weight of (almotriptan malate):(mannitol) is from 1:3 to 1:4.5.
Clause 27.- The pharmaceutical composition according to any one of the preceding clauses, wherein the ratio by weight of (almotriptan malate):(mannitol) is from 1:3.5 to 1:4.0.
Clause 28.- The pharmaceutical composition according to any one of the preceding clauses, wherein the pharmaceutical composition further comprises at least a diluent, preferably in an amount from 2 to 20 % w/w in respect of the total amount of the pharmaceutical composition, more preferably from 7 to 15 % w/w.
Clause 29.- The pharmaceutical composition according to any one of the preceding clauses, wherein the pharmaceutical composition further comprises microcrystalline cellulose, preferably in an amount from 2 to 20 % w/w in respect of the total amount of the pharmaceutical composition, more preferably from 7 to 15 % w/w.
Clause 30.- The pharmaceutical composition according to any one of the preceding clauses, wherein the pharmaceutical composition further comprises at least a lubricant, preferably in an amount from 0.1 to 3 % w/w in respect of the total amount of the pharmaceutical composition, more preferably from 0.5 to 1.5 % w/w in respect.
Clause 31.- The pharmaceutical composition according to any one of the preceding clauses, wherein the pharmaceutical composition further comprises sodium stearyl fumarate, preferably in an amount from 0.1 to 3 % w/w in respect of the total amount of the pharmaceutical composition, more preferably from 0.5 to 1.5 % w/w.
Clause 32.- The pharmaceutical composition according to any one of the preceding clauses, wherein the pharmaceutical composition further comprises at least a binder, preferably in an amount from 1 to 8 % w/w in respect of the total amount of the pharmaceutical composition, more preferably from 2 to 5 % w/w.
Clause 33.- The pharmaceutical composition according to any one of the preceding clauses, wherein the pharmaceutical composition further comprises polyvinylpyrrolidone, preferably in an amount from 1 to 8 % w/w in respect of the total amount of the pharmaceutical composition, more preferably from 2 to 5 % w/w
Clause 34.- The pharmaceutical composition according to any one of the preceding clauses, wherein the pharmaceutical composition further comprises at least a glidant, preferably in an amount from 2 to 8 % w/w in respect of the total amount of the pharmaceutical composition, more preferably from 3 to 6 % w/w in respect of the total amount of the pharmaceutical composition.
Clause 35.- The pharmaceutical composition according to any one of the preceding clauses, wherein the pharmaceutical composition further comprises sodium carboxymethyl starch, preferably in an amount from 2 to 8 % w/w in respect of the total amount of the pharmaceutical composition, more preferably from 3 to 6 % w/w in respect of the total amount of the pharmaceutical composition.
Clause 36.- The pharmaceutical composition according to any one of the preceding clauses, wherein the tablet is a film coated tablet, preferably the amount of film coating ranges from 1 to 5 % w/w.
Clause 37.- The pharmaceutical composition according to any one of the preceding clauses, wherein the water content of the pharmaceutical composition is lower than 4 % w/w measured by loss on drying.
Clause 38.- The pharmaceutical composition according to any one of the preceding clauses, wherein the water content of the pharmaceutical composition is lower than 2 % w/w measured by loss on drying.
Clause 39.- The pharmaceutical composition according to any one of the preceding clauses, wherein the tablet has a hardness of less than 70 N.
Clause 40.- The pharmaceutical composition according to any one of the preceding clauses, wherein the tablet has a hardness from 30 to 55 N.
Clause 41.- The pharmaceutical composition according to any one of the preceding clauses, wherein the tablet has a hardness from 35 to 45 N.
Clause 42.- A blister comprising at least a unit dosage form of a pharmaceutical composition according to any one of the preceding clauses.
Clause 43.- The blister comprising a unit dosage of the pharmaceutical composition according to the preceding clause, wherein the blister is aluminium/PVC blister.
Clause 44.- A pharmaceutical powder comprising almotriptan malate and mannitol, wherein the mannitol is in crystalline form and the bulk density of the mannitol is below 0.75 g/ml.
Clause 45.- The pharmaceutical powder according to the preceding clause, wherein the ratio by weight of (almotriptan malate):(mannitol) is from 1:1 to 1:10.
Clause 46.- The pharmaceutical powder according to any one of the two preceding clauses, wherein the ratio by weight of (almotriptan malate):(mannitol) is from 1:2 to 1:7.
Clause 47.- The pharmaceutical powder according to any one of the three preceding clauses, wherein the ratio by weight of (almotriptan malate):(mannitol) is from 1:3 to 1:4.5.
Clause 48.- The pharmaceutical powder according to any one of the four preceding clauses, wherein the ratio by weight of (almotriptan malate):(mannitol) is from 1:3.5 to 1:4.0.
Clause 49.- The pharmaceutical powder according to any one of the five preceding clauses, wherein the pharmaceutical powder is uniformly distributed.
Clause 50.- A granulate comprising almotriptan malate and mannitol, wherein the mannitol is in crystalline form and the bulk density of the mannitol used for the manufacture the granules is below 0.75 g/ml.
Clause 51.- Almotriptan malate having an X-Ray powder diffraction pattern with peaks at 15.4, 16.2 and 22.1° 2θ (±0.2° 2θ) and wherein the D50 of the almotriptan malate is higher than 20 microns and the D90 is higher than 50 microns.
Clause 52.- Almotriptan malate having an X-Ray powder diffraction peaks at 15.4, 16.2 and 22.1° 2θ (±0.2° 2θ) and wherein the D50 of the almotriptan malate is higher than 20 microns and the D90 is higher than 50 microns and the almotriptan malate is non-micronized.
Clause 53.- Use of the almotriptan malate according to any one of the two preceding clauses for the manufacture of immediate release tablets.
Clause 54.- Use of the almotriptan malate according to the preceding clause in a process for the manufacture of immediate release tablets, wherein the process does not comprise the micronization of the almotriptan malate.
Clause 55.- A pharmaceutical batch of at least 10,000 units of the pharmaceutical composition according to any one of the preceding product clauses.
Clause 56.- The pharmaceutical batch according to the preceding clause, wherein the amount of almotriptan malate in each unit is from about 17.0 to 18.0 mg. Clause 57.- The pharmaceutical batch according to the preceding clause, wherein the units are packaged in blisters, preferably the blister is an aluminium/aluminium blister or an aluminium/PVC blister.
Clause 58.- The pharmaceutical batch according to any one of the preceding three clauses, wherein the relative standard deviation value of content uniformity is less than 5 %, preferably less than 1 %.
Clause 59.- A cardboard box with patient information leaflet in pack sizes of 2, 3, 4, 6, 7, 9, 12, 14 or 18 units of pharmaceutical composition as defined in any one of the pharmaceutical composition clauses, wherein the units are packaged in blisters, and more preferably the blister is an aluminium/aluminium blister or an aluminium/PVC blister.
Clause 60.- A process for the preparation of a pharmaceutical composition comprising almotriptan malate according to any one of the preceding pharmaceutical compositions clauses by wet granulation.
Clause 61.- A process for the preparation of a pharmaceutical composition comprising almotriptan malate according to any one of the preceding pharmaceutical compositions clauses by direct compression.
Clause 62.- A process for the preparation of a pharmaceutical composition comprising almotriptan malate according to any one of the preceding pharmaceutical compositions clauses by dry granulation.
Clause 63.- A process for the preparation of a pharmaceutical composition comprising almotriptan malate according to any one of the preceding pharmaceutical compositions clauses, wherein the process comprises at least the following step:
   i) forming an uniformly distributed powder blend comprising at least almotriptan malate and mannitol, wherein the mannitol is in crystalline form and the bulk density of the mannitol is below 0.75 g/ml.
Clause 64.- The process according to the preceding clause, further comprising the following step:
   ii) wet granulating of the powder blend with a mixture of the binder and water to form granules.
Clause 65.- The process according to the preceding clause, further comprising the following step:
   iii) drying the granules to remove the volatiles, preferably until the water content of the granules is below 2.5 % w/w measured by loss on drying.
Clause 66.- The process according to the preceding clause, further comprising the following step:
   iv) blending the granules with the extragranular excipients, preferably a diluent and a glidant.
Clause 67.- The process according to the preceding clause, further comprising the following step:
   v) lubricating the granulate.
Clause 68.- The process according to any one of the 5 preceding clauses, further comprising the following step:
   vi) compressing the uniformly distributed powder or the granules into a tablet form.

### DESCRIPTION OF THE FIGURES

Figure 1 depicts an X-Ray powder diffraction pattern of almotriptan malate useful for the pharmaceutical compositions as herein disclosed.

### EXAMPLES

### Example 1. Process to obtain almotriptan D,L-hydrogen malate

The almotriptan base (780 g) is dissolved in 1.2 L of MeOH at 40 °C and added dropwise to a solution of 312 g of D,L-malic acid in 2.34 L of MeOH, heated up to 40 °C. The mixture is left to slowly cool down to 20 °C and is stirred for another hour. The separated crystals are filtered and washed with MeOH. Yield: 1014 g (93 %), HPLC purity 99.9 %, melting temp. = 169-171 °C.

### Example 2: X-Ray Powder diffraction

Almotriptan D,L-hydrogen malate crystalline form was obtained following the process described in the state of art ES 2 084 560 B1 or WO 2010113183 A2 and was characterized by X-Ray Powder diffraction.

X-Ray powder diffraction was performed in a Bruker D8 Advance diffractometer with a θ:2θ configuration and Bragg-Brentano geometry with a copper anode tube. The diffractograms were obtained for 2θ angles ranging from 3° to 70° with a step of 0.03° each second. The tube set-up were 40 kV and 30 mA, incident-beam divergence-limiting slit 12 mm, static sample, diffracted-beam receiving slit 0.2 mm and nickel filter.

**Table 1: List of selected peaks obtained by powder X-Ray diffraction of almotriptan D,L-hydrogen malate (see Figure 1).**

| Angle 2θ | Intensity |
|---|---|
| 10.69 | 2,509 |
| 12.27 | 788 |
| 15.43 | 5,192 |
| 15.70 | 5,573 |
| 16.21 | 7,240 |
| 16.54 | 3,676 |
| 18.27 | 2,640 |
| 19.64 | 3,300 |
| 21.19 | 5,579 |
| 22.05 | 16,481 |
| 23.30 | 5,002 |
| 24.71 | 2,854 |
| 26.17 | 3,230 |

### Example 3:

Composition of the coated tablet:

| Active ingredient | | |
|---|---|---|
| | Almotriptan D,L-hydrogen malate | 17.5 mg |

| Excipients | | |
|---|---|---|
| | Mannitol (Pearlitol® 50C) | 66.0 mg |
| | Microcrystalline Cellulose (Avicel® PH 102) | 10.0 mg |
| | Polyvinylpyrrolidone (Plasdone® K29-32) | 3.5 mg |
| | Sodium carboxymethyl starch (Primojel®) | 4.8 mg |
| | Sodium stearyl fumarate | 1.2 mg |
| *Core weight:* | | 103.0 mg |

| Coating | | |
|---|---|---|
| | Opadry white | 3.0 mg |
| *Total weight coated tablet:* | | 106.0 mg |

Manufacturing Process (batch size: 100,000 units):
1. Almotriptan D,L-hydrogen malate, mannitol, and a half of the amount of the sodium carboxymethyl starch were sifted independently through a 1.2 mm screen.
2. The sifted material was mixed from 2 to 5 minutes in the granulation machine (pre-mix).
3. The binding solution (consisting of water and polyvinylpirrolidone) was added and was mixed for approximately 5 minutes.
4. The mixture of components obtained in step 3 was granulated for 3 minutes.
5. The material of step 4 was sifted.
6. The granulate obtained in step 5 was dried until the water content is less than 2 % w/w measured by loss on drying.
7. The dried granulate was fragmented through a 0.8 mm screen.
8. The microcrystalline cellulose and the remaining of sodium carboxymethyl starch were sifted through a 0.8 mm screen and then it was blended these ingredients with the dried granulate for 25 minutes in a V- Blender.
9. Sodium stearyl fumarate was sifted and it was added to the final mix of step 8 and blended for 3 minutes.
10. The lubricated granulate was compressed into tablets using 6 mm diameter cylindrical biconcave punches. The machine was adjusted to obtain tablets of 103.0 mg and 40 N hardness.
11. The uncoated tablets were coated using a Opadry in perforated pan coater equipment.

### Example 4:

Composition of the coated tablet:

| | | |
|---|---|---|
| Active ingredient | | |
| | Almotriptan D,L-hydrogen malate | 17.5 mg |

| Excipients | | |
|---|---|---|
| | Mannitol (Pearlitol® 75C) | 66.0 mg |
| | Microcrystalline Cellulose (Avicel® PH 102) | 10.0 mg |
| | Polyvinylpyrrolidone (Plasdone® K29-32) | 3.5 mg |
| | Sodium carboxymethyl starch (Primojel®) | 4.8 mg |
| | Sodium stearyl fumarate | 1.2 mg |
| *Core weight:* | | 103.0 mg |

| Coating | | |
|---|---|---|
| | Opadry white | 3.0 mg |
| *Total weight coated tablet:* | | 106.0 mg |

The tablets are manufactured as in example 3.

### Example 5:

Composition of the coated tablet:

| Active ingredient | | |
|---|---|---|
| | Almotriptan D,L-hydrogen malate | 17.5 mg |

| Excipients | | |
|---|---|---|
| | Mannitol (Pearlitol® 160C) | 66.0 mg |
| | Microcrystalline Cellulose (Avicel® PH 102) | 10.0 mg |
| | Polyvinylpyrrolidone (Plasdone® K29-32) | 3.5 mg |
| | Sodium carboxymethyl starch (Primojel®) | 4.8 mg |
| | Sodium stearyl fumarate | 1.2 mg |
| *Core weight:* | | 103.0 mg |

| Coating | | |
|---|---|---|
| | Opadry white | 3.0 mg |
| *Total weight coated tablet:* | | 106.0 mg |

The tablets are manufactured as in example 3.

**Table 2. Dissolution profile at pH=6.8 of the batch #12050 (tablets manufactured according to example 3).**

| (min) | 0 | 5 | 10 | 15 |
|---|---|---|---|---|
| #1 | 0.0 | 47.8 | 90.5 | 97.9 |
| #2 | 0.0 | 54.8 | 97.6 | 99.3 |
| #3 | 0.0 | 50.5 | 93.7 | 95.9 |
| #4 | 0.0 | 45.8 | 90.6 | 95.4 |
| #5 | 0.0 | 50.8 | 93.0 | 96.8 |
| #6 | 0.0 | 48.4 | 93.1 | 99.1 |
| #7 | 0.0 | 49.7 | 91.0 | 98.5 |
| #8 | 0.0 | 46.8 | 93.9 | 97.4 |
| #9 | 0.0 | 42.6 | 88.0 | 97.0 |
| # 10 | 0.0 | 31.6 | 76.3 | 94.5 |
| #11 | 0.0 | 39.4 | 89.5 | 95.1 |
| # 12 | 0.0 | 47.0 | 90.1 | 96.3 |
| mean | 0.0 | 46.3 | 90.6 | 96.9 |
| CV (%) | - | 13.1 | 5.7 | 1.6 |

**Table 3. Dissolution profile at pH=6.8 of the batch #12051 (tablets manufactured according to example 3).**

| (min) | 0 | 5 | 10 | 15 |
|---|---|---|---|---|
| # 1 | 0.0 | 53.2 | 92.3 | 96.9 |
| #2 | 0.0 | 49.7 | 94.2 | 97.8 |
| #3 | 0.0 | 49.8 | 93.8 | 96.9 |
| #4 | 0.0 | 39.6 | 85.0 | 96.6 |
| #5 | 0.0 | 48.1 | 88.1 | 96.2 |
| #6 | 0.0 | 36.6 | 70.5 | 90.4 |
| #7 | 0.0 | 46.9 | 90.7 | 96.6 |
| #8 | 0.0 | 43.4 | 87.5 | 95.5 |
| #9 | 0.0 | 39.2 | 80.1 | 95.6 |
| # 10 | 0.0 | 42.3 | 84.7 | 97.3 |
| #11 | 0.0 | 41.0 | 83.3 | 98.6 |
| #12 | 0.0 | 41.4 | 87.8 | 101.8 |
| mean | 0.0 | 44.3 | 86.5 | 96.7 |
| CV (%) | - | 11.6 | 7.7 | 2.7 |

**Table 4. Dissolution profile at pH=6.8 of the batch SPS27001 (tablets manufactured according to example 3).**

| (min) | 0 | 5 | 10 | 15 |
|---|---|---|---|---|
| #1 | 0.0 | 52.3 | 88.3 | 97.5 |
| #2 | 0.0 | 45.6 | 86.0 | 103.7 |
| #3 | 0.0 | 49.5 | 84.7 | 99.9 |
| #4 | 0.0 | 55.9 | 94.6 | 102.3 |
| #5 | 0.0 | 48.8 | 88.1 | 102.6 |
| #6 | 0.0 | 51.4 | 92.9 | 101.3 |
| #7 | 0.0 | 53.5 | 86.7 | 95.6 |
| #8 | 0.0 | 47.5 | 83.2 | 94.6 |
| #9 | 0.0 | 36.2 | 79.6 | 95.7 |
| # 10 | 0.0 | 48.5 | 87.1 | 95.8 |
| #11 | 0.0 | 43.2 | 78.9 | 93.0 |
| # 12 | 0.0 | 45.0 | 80.4 | 90.8 |
| Mean | 0.0 | 48.1 | 85.9 | 97.7 |
| CV (%) | - | 10.9 | 5.7 | 4.2 |

The particle size distribution of the almotriptan D,L-hydrogen malate used in the examples was as follows D50 = 92.9 microns and D90 = 243.8 microns.

### Example 6. Stability Studies for Batch SPS27001 (tablets manufactured according to example 3) in aluminium/PVC blister

Tablets of example 3 were packaged in blisters packs of aluminium/PVC and were subjected to stability tests at times 0; 2 months at 25 °C and 60 % Relative Humidity (RH); 2 months at 30 °C and 65 % Relative Humidity (RH); 2 months at 30 °C and 75 % RH; 2 months at 40°C and 75 % RH.

| | **t=0** | **25 °C / 60% RH - 2 months** | **30 °C / 65% RH - 2 months** | **30 °C / 75% RH - 2 months** | **40 °C / 75% RH - 2 months** |
|---|---|---|---|---|---|
| **Appearance** | film-coated white tablet | film-coated white tablet | film-coated white tablet | film-coated white tablet | film-coated white tablet |
| **Assay (%)** | 102.5 | 98.7 | 98.7 | 100.3 | 98.7 |
| **Disgregation (min)** | 4-6 | 3-4 | 3-4 | 3-4 | 2-5 |
| **RELATED SUBSTANCES** Unknown individual impurities (%) | <0.05 % | <0.05 % | <0.05 % | <0.05 % | <0.05 % |
| **Total impurities (%)** | <0.05 % | <0.05 % | <0.05 % | 0.05 % | 0.09 % |

### Example 7. Stability Studies for Batch SPS27001 (tablets manufactured according to example 3) in aluminium/aluminium blister

Tablets of example 3 were packaged in blisters packs of aluminium/aluminium and were subjected to stability tests at times 0; 2 months at 25 °C and 60 % Relative Humidity (RH); 2 months at 30 °C and 65 % Relative Humidity (RH); 2 months at 30 °C and 75 % RH; 2 months at 40 °C and 75 % RH.

| | **t=0** | **25 °C / 60% RH-2 months** | **30 °C / 65% RH-2 months** | **30 °C / 75% RH-2 months** | **40 °C / 75% RH-2 months** |
|---|---|---|---|---|---|
| **Appearance** | film-coated white tablet | film-coated white tablet | film-coated white tablet | film-coated white tablet | film-coated white tablet |
| **Assay (%)** | 102.5 | 100.4 | 100.5 | 102.0 | 102.4 |
| **Disgregation (min)** | 4-6 | 4-6 | 4-5 | 4-5 | 4-5 |
| **RELATED SUBSTANCES** Unknown individual impurities (%) | <0.05 | <0.05 | <0.05 | <0.05 | <0.05 |
| **Total impurities (%)** | <0.05 | <0.05 | <0.05 | 0.06 | <0.05 |

Unless otherwise indicated, all the analysis methods are carried out according to the European Pharmacopoeia 7th edition.

## Claims

1. A pharmaceutical composition in the form of an immediate release tablet comprising almotriptan malate and mannitol, preferably a film coated tablet, wherein the mannitol is in crystalline form and the bulk density of the mannitol is below 0.75 g/ml.

2. The pharmaceutical composition according to the preceding claim, wherein the bulk density of the mannitol ranges from 0.40 to 0.67 g/ml, preferably from 0.48 to 0.62 g/ml, more preferably from about 0.52 to about 0.58 g/ml, and even more preferably is about 0.55 g/ml; or wherein the mannitol has a mean particle size measured by laser light diffraction from 15 to 180 microns, preferably from 35 to 80 microns.

3. The pharmaceutical composition according to any one of the preceding claims: wherein the almotriptan malate is non-micronized, preferably a non-micronized crystalline form; wherein the D50 of the almotriptan malate is higher than 20 microns, preferably is less than 250 microns, more preferably is between 30 and 190 microns, even more preferably is between 50 and 150 microns, yet even more preferably is between 65 and 135 microns and most preferably is between 80 and 115 microns; and/or wherein the D90 of the almotriptan malate is higher than 50 microns, preferably is less than 700 microns, more preferably is between 80 and 550 microns, even more preferably is between 110 and 475 microns, yet even more preferably is between 150 and 390 microns, most preferably is between 200 and 300 microns.

4. The pharmaceutical composition according to any one of the preceding claims, wherein the almotriptan malate is in crystalline form; or wherein the crystalline form of almotriptan malate shows an X-Ray powder diffraction pattern with peaks at 15.4, 16.2 and 22.1° 2θ (±0.2° 2θ), preferably at 10.7, 15.4, 16.2, 18.3, 19.6 and 22.1° 2θ (±0.2° 2θ), more preferably at 10.7, 15.4, 16.2, 18.3, 19.6 and 22.1° 2θ (±0.2° 2θ), and even more preferably the crystalline form of almotriptan malate has an X-Ray powder diffraction pattern as depicted in figure 1.

5. The pharmaceutical composition according to any one of the preceding claims, wherein the ratio by weight of (almotriptan malate):(mannitol) is from 1:1 to 1:10, preferably is from 1:2 to 1:7, more preferably is from 1:3 to 1:4.5, and even more preferably is from 1:3.5 to 1:4.0.

6. The pharmaceutical composition according to any one of the preceding claims: wherein the pharmaceutical composition further comprises at least a diluent, preferably in an amount from 2 to 20 % w/w, more preferably from 7 to 15 % w/w; wherein the pharmaceutical composition further comprises microcrystalline cellulose, preferably in an amount from 2 to 20 % w/w, more preferably from 7 to 15 % w/w; wherein the pharmaceutical composition further comprises at least a lubricant, preferably in an amount from 0.1 to 3 % w/w, more preferably from 0.5 to 1.5 % w/w ; wherein the pharmaceutical composition further comprises sodium stearyl fumarate, preferably in an amount from 0.1 to 3 % w/w, more preferably from 0.5 to 1.5 % w/w; wherein the pharmaceutical composition further comprises at least a binder, preferably in an amount from 1 to 8 % w/w, more preferably from 2 to 5 % w/w; wherein the pharmaceutical composition further comprises polyvinylpyrrolidone, preferably in an amount from 1 to 8 % w/w, more preferably from 2 to 5 % w/w; wherein the pharmaceutical composition further comprises at least a glidant, preferably in an amount from 2 to 8 % w/w, more preferably from 3 to 6 % w/w ; or wherein the pharmaceutical composition further comprises sodium carboxymethyl starch, preferably in an amount from 2 to 8 % w/w, more preferably from 3 to 6 % w/w, being all the % w/w in respect of the total amount of the pharmaceutical composition.

7. The pharmaceutical composition according to any one of the preceding claims, wherein: the water content of the pharmaceutical composition is lower than 4% w/w measured by loss on drying, preferably is lower than 2% w/w measured by loss on drying; and/or the tablet has a hardness of less than 70 N, preferably from 30 to 55 N, more preferably from 35 to 45 N.

8. A packaged pharmaceutical composition, wherein said packaged pharmaceutical composition is: a blister comprising a pharmaceutical composition as defined in any one of the preceding claims, preferably the blister is aluminium/aluminium blister or aluminium/PVC blister or a cardboard box with patient information leaflet comprising at least one of said blister.

9. A pharmaceutical powder comprising almotriptan malate and mannitol, wherein the mannitol is in crystalline form and the bulk density of the mannitol is below 0.75 g/ml, advantageously the ratio by weight of (almotriptan malate):(mannitol) is from 1:1 to 1:10, preferably is from 1:2 to 1:7, more preferably is from 1:3 to 1:4.5, and even more preferably is from 1:3.5 to 1:4.0, and/or the pharmaceutical powder is uniformly distributed.

10. A granulate comprising almotriptan malate and mannitol, wherein the mannitol is in crystalline form and the bulk density of the mannitol used for the manufacture the granulesl is below 0.75 g/ml.

11. Almotriptan malate: having an X-Ray powder diffraction pattern with peaks at 15.4, 16.2 and 22.1° 2θ (±0.2° 2θ) and wherein the D50 of the almotriptan malate is higher than 20 microns and the D90 is higher than 50 microns; or having an X-Ray powder diffraction pattern with peaks 15.4, 16.2 and 22.1° 2θ (±0.2° 2θ) and wherein the D50 of the almotriptan malate is higher than 20 microns and the D90 is higher than 50 microns and the almotriptan malate is non-micronized.

12. Use of the almotriptan malate according to any one of the two preceding claims for the manufacture of immediate release tablets, preferably the use in a process for the manufacture of said tablets, wherein the process does not comprise the micronization of the almotriptan malate.

13. A pharmaceutical batch of at least 10,000 units of the pharmaceutical composition according to any one of the preceding product claims, preferably: the amount of almotriptan malate in each unit is from about 17.0 to 18.0 mg; and/or wherein the units are packaged in blisters, preferably the blister is an aluminium/aluminium blister or an aluminium/PVC blister.

14. A process for the preparation of a pharmaceutical composition comprising almotriptan malate according to any one of the preceding pharmaceutical composition claims by wet granulation, by direct compression or by dry granulation.

15. A process for the preparation of a pharmaceutical composition comprising almotriptan malate according to any one of the preceding pharmaceutical composition claims, wherein the process comprises at least the following step (i):
i) forming an uniformly distributed powder blend comprising at least almotriptan malate and mannitol, wherein the mannitol is in crystalline form and the bulk density of the mannitol is below 0.75 g/ml.
ii) wet granulating of the powder blend with a mixture of the binder and water to form granules.
iii) drying the granules to remove the volatiles, preferably until the water content of the granules is below 2.5 % w/w measured by loss on drying.
iv) blending the granules with the extragranular excipients, preferably a diluent and a glidant.
v) lubricating the granulate.
vi) compressing the uniformly distributed powder or the granules into a tablet form.
